# EUROPEAN PATENT APPLICATION

(11) **EP 1 611 904 A1**
(43) Date of publication of application: **04.01.2006**
(21) Application number: 05076224.4
(22) Date of filing: 25.05.2005
(51) Int. Cl.: A61L 9/12

(54) **Air Freshener**

(30) Priority: 28.06.2004 IT re20040074
(71) Applicant: RE.LE.VI. S.p.a., 46040 Rodigo (Mantova) (IT)
(72) Inventor: Pagani, Fabio, 46040 Rodigo (Mantova) (IT)
(74) Representative: Corradini, Corrado

(57) **Abstract**

The air freshener comprises a container (2) for a perfuming substance, a cap (3) having a cup configuration for screwing on the neck (20) of the container, and a wick-holder frame (4) that is inserted by sliding inside the container (2) and removably attached to the cap (3). The cap (3) comprises at least two component elements (31, 32) for reciprocal assembly as a hermetic closure, where a first component element (31) has a through passage cavity generally cylindrical and it is intended for insertion into the neck of the container (2), and a second component element (32) is intended to be removably joined with the first component element (31) to close the through passage cavity in a hermetic manner, said two component elements (31, 32) being structured in such a manner to provide a check seat with bilateral engagement which is adapted to block axially but not torsionally, the curved hook means (16) foreseen in the upper part of the frame. When the dispenser is prepared the first component element (31) of the cap is screwed onto the neck (20) of the empty container (2), followed by the insertion of the frame (4) mounted with the wick (5) inside the container, with the engagement of the top of the frame with the upper hooking throat applied by said first component element (31); The container is then filled with the perfuming substance, and the second component element (32) is coupled with the top of the first component element(31) thus simultaneously providing for the closure of the container and ensuring a block system to prevent the frame (4) below from sliding.

## Description

The present finding refers to air fresheners for internal environments that contain a perfuming aromatic substance or product (perfuming and/or deodorising) generally liquid, adapted to evaporate automatically when exposed to air.

The finding also intends protecting a method for the preparation for use of an improved dispenser in accordance with the teachings of the present invention.

Dispensers for air freshening in enclosed environments in general, such as domestic or public areas, have been known for some time, these generally comprising a container destined to contain a volatile perfuming substance, generally liquid, a screw top cap destined to open/close said container, and a hydrophilic element associated with said cap to expose the perfuming substance to the air when the cap is open.

In known dispensers of this kind, the said container generally has a bottle form with a threaded neck, the said screw cap generally having the form of a synthetic monolithic body with an inverted cup configuration, threaded internally, and said hydrophilic element generally having the form of a strip of cloth or non-woven material or some other equivalent material able to absorb said perfuming aromatic volatile substance.

Said hydrophilic element, that for the sake of simplicity will be hereafter referred to as a wick, is removably associated with the cap by means of a small flat synthetic frame, generally very thin and having an elongated shape adapted for insertion inside the respective container, also having a length that is less than the depth or overall height of said container.

Said small frame presents openings or notches that permit the wick to be wound around along the whole length of the frame, said wick having an extra section that extends from the frame at the opposite end from the cap, in a manner that maintains the wick constantly dampened even when the cap is open and in raised position, wherein the wick exposes to the air the perfuming substance in which it is immersed in the above described manner.

In similar known dispensers, the system that connects the cap and frame is generally a snap-on connection comprising a male element extending from the internal side of the top of the cap (or the top of the frame) and a connected female element associated with the top of the frame (or the internal side of the top of the cap).

Said male and female elements can assume different forms, for example the male element can be composed of an enlarged head set on the free end of a rod, and the female element can be an impression with an undercut aligned with the mouth opening.

In particular reference to the element, whether male or female, associated with said frame, it should be stated that it is attached to the upper rim of the frame, where said rim is composed of the upper side or edge of an opening underneath it, adapted for the passage of the wick, which once installed on the frame is supported by the lower side or edge of said opening.

Known dispensers such as those described also normally comprise a narrowing section provided at the base of the container's neck, and which performs various functions.

One function in the case of careless detaching of the wick holder frame from the cap, is to prevent the wick-holder frame from falling into the bottom of the container, and this is performed by protruding support members arranged on the frame that engage with the narrowed section; another function is to act as a corresponding engagement for said members in order to provoke the automatic activation of said snap-on connection by means of the simple screwing of the cap on the neck of the container, for example in the above theoretical situation of careless detaching of the frame from the cap; and yet a further function is a necking action to block the wick-holder frame in working position where it is partially extracted from the container and is generally supported by the existing engagement between said wick and said necking, and where, thanks to the said necking effect, the perfuming substance present in the container, i.e. the substance beneath the narrowed necking level, is prevented from evaporating. This necking also acts to squeeze the wick to prevent the upper part from being overly soaked in the substance.

Air fresheners for enclosed environments of the type described above present certain drawbacks.

Firstly the presence of said opening foreseen at the top of the wick-holder frame and envisaged to provide a through passage for the wick, makes the installation of the wick on the frame relatively lengthy and complex, and therefore relatively expensive, because in order to be wound around the frame the wick must necessarily be inserted at least once through said opening.

Furthermore, the said upper side or edge of said opening could create difficulties or obstacles when filling the container during dispenser production or preparation for use.

Secondly the presence of the above snap-on connection between the frame and the cap often leads to undesired accidental detachment from one another, in particular, when the container is opened, especially if the frame and container are not maintained practically in a coaxial position.

The main aim of the present finding is to provide an improved dispenser able to overcome the problems described above.

Another aim of the finding is to achieve the said result within the context of a simple, rational, reliable, robust and economical constructive solution, that is also easy to assemble.

The aforesaid aims are achieved thanks to the characterising elements described in the main claim.

Preferred and advantageous embodiments of the proposed means are defined in the depending claims.

Generally speaking, the air fresheners according to the finding is of the type described in the introduction, that is it comprises a container for an aromatic substance, generally liquid, a cap in inverted cup form adapted to be screwed onto the neck of said container, and a wick-holder frame that is removably attached to said cap.

In particular, according to the teachings of this invention it is foreseen that the cap will be realised in more than one component elements that can be reciprocally assembled together, typically two.

In more detail, one of said two component elements presents a through passage cavity, generally cylindrical, and intended to be inserted in the neck of the container, and the other component element is intended to be joined with the first element, after the installation of the wick-holder frame inside the container, where it closes the upper opening of said through passage cavity hermetically, and cooperates with said element of the cap to create a check seat with bilateral engagement to block axially but not by torsion, special fixing means provided in the upper area of the frame.

Said first component element can be conveniently composed of a tubular element, said second component element can be conveniently composed of a generally disk shaped element to act as a closure for the upper mouth opening of said cylindrical element, and said cylindrical and disk-shaped elements can be conveniently united by means of a snap-on pressure type block system.

All the aims of the finding are achieved by the means described above.

In fact, the provision of a modular cap according to the finding permits the construction of the frame in a manner to present an upper slot, or recess open at the top so that the winding of the wick at the top of the frame presents no problems, just as there are no obstacles or impediments when filling the container with the wick-holder frame previously inserted.

Furthermore, said axial block seat provided by the modular cap according to the finding, eliminates any risk of accidental detachment of the wick-holder frame from the cap during cap manipulation, in particular during the opening of the container or the extraction of the frame.

The characteristics and constructive benefits of the dispenser in question, as well as the preparation method thereof will be made clear from the detailed description below, with references to the figures in the appended drawings providing examples wherein:
FIGURE 1 is an axial section view showing the dispenser according to the finding in exploded configuration and with interruptions.
FIGURE 2 is the section II-II shown in FIG.1.
FIGURE 3 is an elevation view showing the frame complete with wick.
FIGURE 4 is a view similar to that shown in FIG.1 showing the upper part of the dispenser in enlarged scale in its closed configuration.
FIGURE 5 is a view similar to that shown in FIG.2 showing the upper part of the dispenser in enlarged scale in its closed configuration.
FIGURE 6 is a view similar to that shown in FIG.4 showing the upper part of the dispenser in a use configuration.

The illustrated dispenser is basically composed of a container 2, a cap 3 to close said container 2, a frame 4, and a generally hydrophilic element or wick 5.

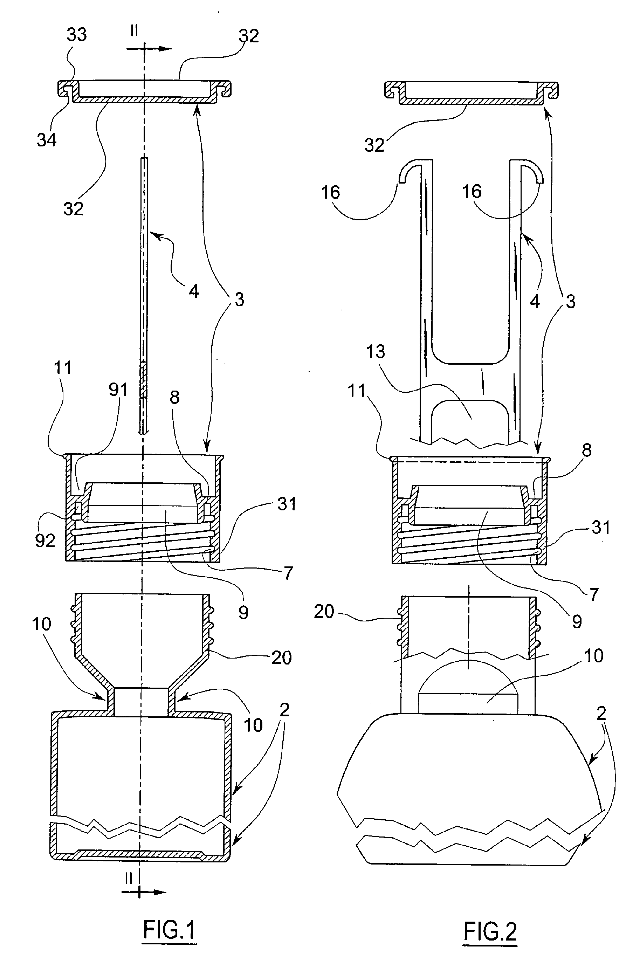

Container 2 is composed of a suitable material such as a synthetic material and can assume any form whatsoever, such as a bottle, flask, (as in fig.2) or jar.

Said container 2 is intended to receive a volatile perfuming substance, generally liquid, known per se, and not shown for simplicity.

The same container 2 presents a neck 20 with external threading, at the base of which is a narrowing or necking (See Figs 1, 2 and 4) provided by two identical transverse recesses diametrically opposite 10, foreseen in container 2.

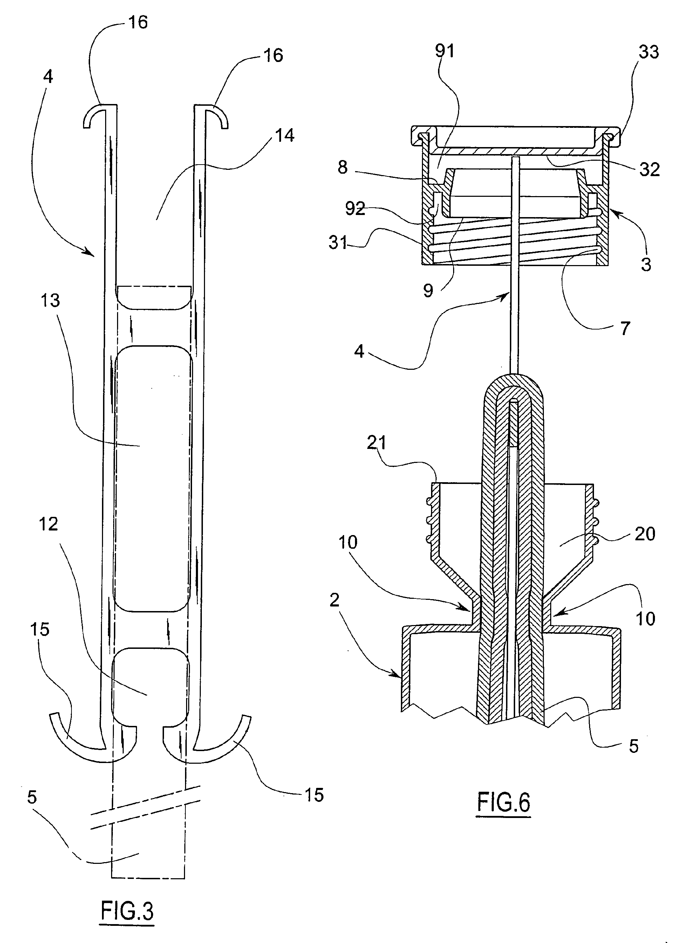

The function of said narrowing is to squeeze the wick 5 when the container is open as shown in fig.6, where it supports wick 5 and frame 4, and practically closes the existing gap between the said recesses 10 to prevent the perfuming substance under the narrowed portion from evaporating, and also to squeeze the wick to prevent the upper portion from being overly soaked in the perfuming substance.

In accordance with the finding, cap 3 is composed of two component elements 31 and 32 that can be reciprocally assembled, and that are composed of a suitable material, typically a synthetic material.

The first component 31 comprises a tubular element with a vertical axis having a through passage cavity, and the second element 32 comprises a solid generally flat body set coaxially with the first element 31.

In particular, said tubular element 31 comprises a sleeve inside which exists a thread 7 for screwing onto the threading on neck 20 of the container 2, in the lower area, and a bracket around the total circumference 8, whose internal rim bears a ring 9, in the middle area.

The latter is composed of a lower cylindrical strip and an upper frusto-conical strip tapering towards the top, where both form in combination with said sleeve 31 and said bracket 8 respective ring shaped throats 91 and 92.

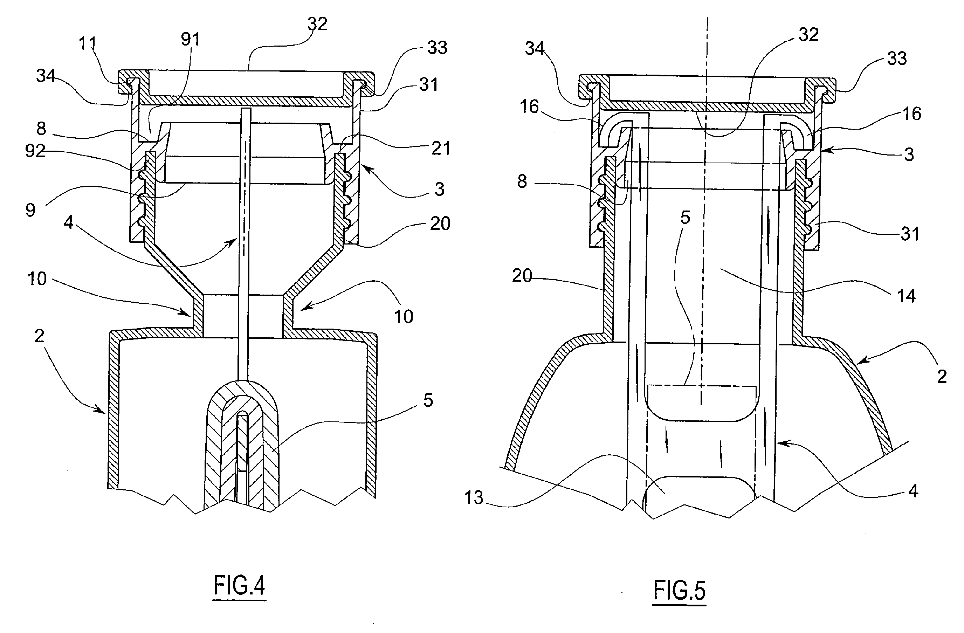

The lower throat 92 provides a seat to receive the upper mouth 21 of the neck 20 of the container 2 (See figs. 4 and 5), and the upper throat 91 provides a seat to attach the frame 4 (Figs. 4 to 6).

Furthermore, the total upper external circumferential rim of sleeve 31 has a ribbing 11 suitably arched transversally.

The second component 32 of cap 3 acts as a closure for the upper mouth of the cavity of sleeve 31, and is generally configured as a flat cover.

In particular, said closure 32 has a relatively thin disk-shaped form with an external circumferential rim bent over successively upwards, then outwards, and then downwards to provide a ring-shaped throat 33 facing downwards for assembly with the upper circumferential rim of sleeve 31.

Furthermore, along the whole lower internal circumferential rim of the external wall of said throat 33 runs a lip 34 intended to act as a snap-on connection under the upper external ribbing 11 of said sleeve 31.

In particular, said ribbing 11 and said lip 34 are shaped and dimensioned in such a manner to ensure that the snap-on connection thereof is reasonably tight or secure in order to create a hermetic union without the need for an interposed special means such as a seal.

Frame 4 comprises a flat element relatively fine in thickness (Figs. 1,4, and 6) in elongated form (Fig. 3) composed of a suitable material, typically a synthetic material.

The actual body of said flat element presents, when laid flat, the form of a vertical band whose lesser side or base is practically the same size as the lesser diameter of the frusto-conical strip of the ring 9 described above, and whose larger side or height is lesser than the depth or height of container 2.

Said band shaped body presents (see detail in fig.3) in its lower part a recess 12 open towards the bottom, and in its intermediate part a rectangular shaped opening 13, and in its upper part a straight seat 14 open towards the top.

At the lower end of the frame 4 (see fig.3) are two identical curved elements on opposite sides 15 that extend from the sides of frame 4 and curve in an upward direction to prevent the frame 4 from sliding out from container 2.

Furthermore, at the top end of the frame 4 are two identical hook-shaped elements 16 set on opposite sides that extend from the sides of the frame 4, set on the same plane as the frame, and curved in a downward direction.

As regards wick 5, it comprises a strip of hygroscopic material such as cloth, non-woven fabric or some other equivalent hydrophilic material.

Said strip has a width similar to that of the seat 14 of frame 4 and a length greater than that of frame 4, in order to allow it to be wrapped completely around the length of the frame at least once as shown in figs. 4 and 6.

In particular, when the wick 5 is inserted on the frame 4, one end of wick 5 is blocked inside the recess 12, and the other end of the wick 5, after a return passage on the bottom of seat 14, will be left to hang (Fig.3) so that it remains immersed in the perfuming substance even when the container 2 is open. (Fig.6).

Once the wick 5 has been inserted on the frame 4, the dispenser is assembled as follows:
The empty container 2 is moved to a suitable workstation, and the sleeve 31 is screwed into the container as far as possible.
When this is completed, the frame 4 containing wick 5 is inserted from top to bottom in container 2 engaging its hooks 16 with the upper internal ring-shaped throat 91 of sleeve 31.
Alternatively, the top of frame 4 containing the wick 5 can be inserted passing it from bottom to top through the sleeve 31 simultaneously engaging the hooks 16 into the upper throat 91. Then the frame 4 with the wick is inserted into the container and the sleeve 31 is screwed onto the neck 20 of the empty container 2.
At this point the perfumed substance is introduced into the container 2, and lastly the closure 32 is pressed onto sleeve 31 in a snap-on action to form a hermetic closure.
The hooks 16 and the surrounding elements of the cap 3 are shaped and dimensioned in such a manner to ensure that when cap 3 is assembled, the cap remains axially engaged with frame 4 on both sides, while permitting mutual rotation of both elements as shown in figures from 4 to 6.
The dispenser is therefore ready for use as shown in figure 6, by unscrewing the cap 3 and by extracting the required portion of frame 4 with the wick 5 from the container 2.

The benefits and advantages of the finding are clearly understandable from the aforesaid description and from the appended figures.

## Claims

1. Air freshener, comprising a container (2) for a perfuming substance, a cap (3) having a cup configuration adapted for screwing on the neck (20) of said container, and a wick-holder frame (4) that is inserted by sliding it inside said container (2) and removably attached to said cap (3), **characterised in that** the cap (3) comprises at least two component elements (31, 32) for reciprocal assembly as a hermetic closure, where a first component element (31) has a through passage cavity generally cylindrical and is intended for insertion into the neck of the container (2), and a second component element (32) is intended to be removably joined with the first component element (31) to close the through passage cavity in a hermetic manner, said two component elements (31, 32) being structured in such a manner to form a non-return seat on both sides intended to block axially but not torsionally, the curved hooked-shaped means (16) foreseen in the upper part of the frame.

2. Air freshener according to claim 1, **characterised in that** said first component element (31) of the cap comprises at the lower end, a tubular body that presents an internal threaded section (7) for connection to the neck (20) of the container (2); in the intermediate position, an internal circumferential bracket (8) that bears a coaxial ring (9), under and over which are defined two ring-shaped throats (92, 91) to receive the upper mouth (21) of the neck (20) of the container and the said hook-shaped means (16) of the frame respectively; and at the top end, the connection seat for said second component element (32) of the cap.

3. Air freshener according to claim 2 **characterised in that** said connection seat comprises circumferential ribbing (11) for the whole perimeter of the external rim of the upper end of said tubular body. (31).

4. Air freshener according to claim 1 **characterised in that** said second component element (32) of the cap (3) comprises a closure whose total circumferential rim bears a throat (33) intended to engage in a snap-on action with said circumferential ribbing (11) of said tubular body (31).

5. Air freshener according to claim 1 **characterised in that** said frame (4) has, in its upper part, a seat (14) facing in an upward direction and open at the top, whose bottom end acts as a return element for the respective wick (5) and whose sides are adapted with said hook-shaped means (16) in the upper part.

6. Air freshener according to claim 5 **characterised in that** said hook-shaped means (16) comprise two projecting curved hooks set on opposite sides intended to connect with the upper internal ring-shaped throat (91) of said component element of the cap in a slightly loose manner, and maintained in said condition by the second component element (32) of the cap.

7. Method for providing a air freshener according to claims 1 to 6 **characterised in that** it comprises the following operational steps:
- Screwing of said first component element (31) of the cap on the neck (20) of the empty container (2).
- Insertion of the frame (4) previously mounted with the relative wick (5) inside the container, simultaneously engaging the top of the frame with the respective upper hooking throat applied by said first component element (31) of the cap,
- Filling of the container with the perfuming substance, and
- Assembly of second component element (32) of the cap with the top of the first component element (31) thus simultaneously providing for the closure of the container and ensuring a block system to prevent the frame (4) below from sliding.

8. Method for providing a air freshener according to claims 1 to 6 **characterised in that** it comprises the following operational steps:
- Insertion of the top of the frame (4) previously mounted with the relative wick (5) through said first component element (31) of the cap, simultaneously engaging the relative curved hooks (16) in the respective upper hooking throat applied by said first component element (31),
- Insertion of the frame (4) containing the wick inside the container and screwing of said first component element (31) onto the neck (20) of the empty container (2),
- Filling of the container with the perfuming substance, and
- Assembly of second component element (32) of the cap with the top of the first component element (31) thus simultaneously providing for the closure of the container and ensuring a block system to prevent the frame (4) below from sliding.
